# EUROPÄISCHE PATENTSCHRIFT

(11) **EP 2 307 356 B1**
(45) Veröffentlichungstag und Bekanntmachung des Hinweises auf die Patenterteilung: **06.06.2012**
(21) Anmeldenummer: 09780956.0
(22) Anmeldetag: 23.07.2009
(51) Int. Cl.: C07C 263/10

(54) **VERFAHREN ZUR HERSTELLUNG VON ISOCYANATEN**
METHOD FOR PRODUCING ISOCYANATES
PROCÉDÉ DE FABRICATION D'ISOCYANATES

(30) Priorität: 23.07.2008 EP 08160990
(43) Veröffentlichungstag der Anmeldung: 13.04.2011
(73) Patentinhaber: BASF SE, 67056 Ludwigshafen (DE)
(72) Erfinder: KNÖSCHE, Carsten, 67150 Niederkirchen (DE); MATTKE, Torsten, 67251 Freinsheim (DE)
(86) Internationale Anmeldenummer: PCT/EP2009/059460
(87) Internationale Veröffentlichungsnummer: WO 2010/010135

(56) Entgegenhaltungen:
- DE-A1- 3 842 065
- US-A1- 2004 192 959
- US-A1- 2005 137 417

## Beschreibung

Die Erfindung betrifft ein Verfahren zur Herstellung von Isocyanaten durch Umsetzung der korrespondierenden Amine mit Phosgen in der Gasphase, gegebenenfalls in Gegenwart mindestens eines Inertmediums, wobei das Phosgen durch einen ersten Einlass und das Amin durch einen zweiten Einlass eines Ejektors in einen Reaktor geleitet werden.

Die Herstellung von Isocyanaten durch Phosgenierung der korrespondierenden Amine kann prinzipiell durch eine Flüssigphasen- oder eine Gasphasenphosgenierung erfolgen. Die Gasphasenphosgenierung zeichnet sich dadurch aus, dass eine höhere Selektivität, ein geringerer Hold-up an toxischem Phosgen sowie ein verminderter Energiebedarf erforderlich sind.

Bei der Gasphasenphosgenierung werden ein aminhaltiger Eduktstrom und ein phosgenhaltiger Eduktstrom jeweils in gasförmigem Zustand vermischt. Das Amin und das Phosgen setzen sich unter Freisetzung von HCl zu den entsprechenden Isocyanaten um. Der aminhaltige Eduktstrom liegt im Allgemeinen in flüssiger Phase vor und muss vor der Vermischung mit dem phosgenhaltigen Strom verdampft und gegebenenfalls überhitzt werden.

Aufgrund des geringen Dampfdrucks, insbesondere der Diamine, erfolgt die Verdampfung bei erhöhter Temperatur. Dadurch werden jedoch auch Zersetzungsreaktionen der Amine bzw. Diamine, beispielsweise Desaminierungen, Demethylierungen, Dimerisierungen, hervorgerufen, die sich negativ auf die Selektivität des Gesamtprozesses auswirken.

Zudem setzen bei der Kontaktierung der beiden Eduktströme aufgrund der hohen Temperaturen schnell reaktive Umsetzungen ein. Neben der Phosgenierung des Amins zum Isocyanat können unerwünschte Neben- und Folgereaktionen einsetzen. So kann zum Beispiel bereits gebildetes Isocyanat mit noch nicht abreagiertem Amin einen Harnstoff bilden. Des Weiteren können auch Carbodiimide und Cyanurate entstehen. Dies wirkt sich zum einen auf die Selektivität des Prozesses aus, zum anderen können gebildete feste Nebenprodukte zu Verstopfungen führen und sich so negativ auf die Laufzeit der Anlage auswirken. Daher ist es im Allgemeinen ein Bestreben, die Eduktströme möglichst schnell zu vermischen, um Mischverhältnisse, die die Bildung von Nebenkomponenten beschleunigen, soweit wie möglich zu vermeiden.

So ist es zum Beispiel aus EP-A 1 319 655 bekannt, zur Herstellung von Diisocyanaten und Triisocyanaten die korrespondierenden, dampfförmigen Di- und/oder Triamine sowie Phosgen getrennt auf Temperaturen im Bereich von 200 °C bis 600 °C zu erhitzen und durch ein statisches Mischorgan zu leiten, wobei die gasförmigen Edukte parallel einen Reaktor durchströmen und das Amin durch eine Düse des Mischorgans und das Phosgen durch einen die Düse umgebenden Ringraum dem Reaktor zugeführt wird. Der Ringraum weist dabei den gleichen Durchmesser auf wie der Reaktor. Die Geschwindigkeit des Amins ist dabei groß gegenüber der Geschwindigkeit des Phosgens.

Aus EP-A 0 928 785 ist es bekannt, dass Amin und dass Phosgen in einem Mikrostrukturmischer zu mischen. In diesem Fall treten die Edukte in Form dünner Freistrahlen aus dem Bauteil aus, wo sie sich dann sehr schnell durch Diffusion und/oder Turbulenz vermischen. Hierdurch wird der Vermischungsvorgang beschleunigt und die erzielbare Ausbeute erhöht.

Die Herstellung von Isocyanaten durch Phosgenierung der korrespondierenden Amine in einem Rohrreaktor, der ein zentrisch in Richtung der Rotationsachse des Rohrreaktors ausgedehntes, doppelwandiges Leitrohr aufweist, ist in EP-A 1 555 258 beschrieben. Zwischen der inneren und der äußeren Wand des doppelwandigen Leitrohrs ist ein konzentrischer Ringspalt ausgebildet. Für die Reaktion werden dampfförmige Diamine und/oder Triamine und Phosgen getrennt voneinander auf Temperaturen im Bereich von 200 °C bis 600 °C erhitzt und anschließend werden die dampfförmigen Diamine und/oder Triamine dem Rohrreaktor über den konzentrischen Ringspalt zugeführt und das Phosgen auf der verbleibenden Querschnittsfläche des Rohrreaktors.

In EP-A 1 526 129 ist ein Verfahren zur Herstellung von Isocyanat beschrieben, bei dem ein Diamine und/oder Triamine enthaltender Eduktstrom einem Rohrreaktor über einen Ringraum, der eine Zentraldüse umgibt, zugeführt wird und ein Phosgen enthaltender Eduktstrom über die Zentraldüse zugeführt wird. Der Ringraum weist dabei den gleichen Durchmesser auf wie der Reaktor. Zur Turbulenzerzeugung ist in der Zentraldüse ein Turbulenzerzeuger angeordnet.

Auch aus EP-A 0 289 840 ist es bekannt, zunächst Phosgen durch eine Düse, die in ein Mischrohr hineinragt, einzuleiten. Durch einen die Düse umgebenden Ringspalt wird ein Amin eingeleitet. Das Phosgen und das Amin werden vor dem Einleiten auf eine Temperatur im Bereich von 200 bis 600 °C, vorzugsweise von 200 bis 500 °C, erhitzt.

Nachteil aller bekannter Verfahren ist es, dass aufgrund der hohen Temperaturen sofort mit der Durchmischung die Reaktion einsetzt und aufgrund des gleichzeitigen Vorhandenseins von Amin und Isocyanat auch korrespondierende Harnstoffe in einer Nebenreaktion gebildet werden.

Aus DE-A 38 42 065 ist es bekannt, zur Verdampfung einer hochsiedenden Substanz einen Ejektor einzusetzen. Durch den Ejektor wird in einer zentralen Düse ein erstes Edukt mit einer hohen Geschwindigkeit geleitet. Hierdurch wird das zweite Edukt aus einem Ringraum mitgerissen. Es entsteht ein Unterdruck im Bereich der Zufuhr des zweiten Edukts zum Reaktor. Aus diesem Grund kann bei einer niedrigeren Temperatur verdampft werden. Dies führt jedoch dazu, dass das den Ejektor verlassende Reaktionsgemisch eine Temperatur aufweist, die unterhalb der geeigneten Reaktionstemperatur liegt.

Aufgabe der vorliegenden Erfindung ist es, ein Verfahren zur Herstellung von Isocyanaten durch Umsetzung der korrespondierenden Amine mit Phosgen in der Gasphase bereitzustellen, das eine schnelle Durchmischung der Edukte und eine weitere Reduktion der Bildung von Nebenprodukten ermöglicht.

Gelöst wird die Aufgabe durch ein Verfahren zur Herstellung von Isocyanaten durch Umsetzung der korrespondierenden Amine mit Phosgen in der Gasphase, gegebenenfalls in Gegenwart mindestens eines Inertmediums, wobei das Phosgen durch einen ersten Einlass und das Amin durch einen zweiten Einlass eines Ejektors in einen Reaktor geleitet werden. Der erste Einlass und der zweite Einlass münden in eine Mischzone, in der das Phosgen und das Amin zu einem Reaktionsgemisch gemischt werden. An die Mischzone schließt sich ein Diffusor an, in dem Druck und Temperatur des Reaktionsgemischs aus Phosgen und Amin erhöht werden.

Das Phosgen tritt vorzugsweise mindestens mit Schallgeschwindigkeit in die Mischzone ein. Hieraus resultiert in der Mischzone eine Geschwindigkeit üblicherweise von mehr als 0,5 Ma, so dass die Temperatur in der Mischzone deutlich niedriger ist als im Reaktor. Zudem ist auch der Druck in der Mischzone niedriger als im Reaktor. Eine Verdampfung des Amins bei niedrigeren Temperaturen wird dadurch erzielt, dass der Druck in der Zuleitung des Amins zur Mischzone gegenüber dem Druck im Reaktor deutlich verringert ist.

Der erste Einlass und der zweite Einlass, die in die Mischzone münden, sind vorzugsweise eine zentrale Düse und ein die zentrale Düse umgebender Ringspalt. In einer bevorzugten Ausführungsform wird das Phosgen durch die zentrale Düse und das Amin durch den die zentrale Düse umgebenden Ringspalt in die Mischzone geleitet. Es ist alternativ jedoch auch möglich, dass das Phosgen durch den die zentrale Düse umgebenden Ringspalt und das Amin durch die zentrale Düse zugegeben werden. Wenn das Phosgen über den die zentrale Düse umgebenden Ringspalt zugegeben wird, ist die Neigung zur Bildung von Ablagerungen geringer als bei der Zugabe des Phosgens über die zentrale Düse. Jedoch ist die Zugabe des Phosgens über den die zentrale Düse umgebenden Ringspalt nachteilig hinsichtlich des Druckverlustes. Üblicherweise wird das Phosgen deshalb über die zentrale Düse zugeführt.

Durch den Ejektor wird eine schnelle Durchmischung des Phosgens mit dem Amin erzielt. Aufgrund der hohen Geschwindigkeit des Phosgens wird die der Düse folgende Mischzone turbulent durchströmt, so dass sich eine Durchmischung aufgrund der Turbulenz ergibt. In dem sich an die Mischzone anschließenden Diffusor werden die Temperatur und der Druck gegenüber der Mischzone erhöht. Dies ermöglicht es, die Durchmischung von Phosgen und Amin in der Mischzone bei einer Temperatur durchzuführen, die unterhalb der Temperatur liegt, bei der die Reaktion mit maximaler Reaktionsgeschwindigkeit abläuft. Gleichzeitig sind auch die Partialdrücke von Phosgen und Amin vermindert. Hieraus resultiert eine geringere Reaktionsgeschwindigkeit in der Mischzone. Aufgrund der geringeren Reaktionsgeschwindigkeit in der Mischzone wird weniger Isocyanat hergestellt. Hierdurch lässt sich die Bildung von Nebenprodukten, insbesondere von Harnstoff, durch die gleichzeitige Anwesenheit von Isocyanat und Amin reduzieren. Der Temperaturanstieg auf die eigentliche Reaktionstemperatur und den Reaktionsdruck erfolgt im Diffusor.

Aufgrund der schnellen Strömung des Phosgens, vorzugsweise durch die zentrale Düse, wird in der Aminzuführung, d.h., bei über die zentrale Düse zugegebenem Phosgen, im die zentrale Düse umgebenden Ringspalt und dem in den Ringspalt mündenden Vorlauf, ein Unterdruck erzeugt. Da die Siedetemperatur eines Stoffes vom Druck abhängig ist und bei niedrigerem Druck auch die Siedetemperatur sinkt, erfolgt die Verdampfung des Amins mit abnehmendem Druck somit bei niedrigeren Temperaturen. Durch die Verdampfung bei niedrigeren Temperaturen kann das Amin schonender verdampft werden.

Der Druck, der in der Aminzuführung und damit im Vorlauf des Amins erzielt werden kann, ist dabei abhängig von der Geschwindigkeit des Phosgens und den geometrischen Abma-βen von Düse und Ringspalt. Mit zunehmender Geschwindigkeit des Phosgens nimmt der Druck im Ringspalt und dem Vorlauf ab.

Geeignete Drücke, die in der Aminzuführung und im Vorlauf des Amins, bevorzugt im Ringspalt und im in den Ringspalt mündenden Vorlauf, erzielt werden, sind im Allgemeinen abhängig vom eingesetzten Amin. Bevorzugt liegt der Druck im Vorlauf des Amins im Bereich von 0,01 bis 3 bara, insbesondere im Bereich von 0,05 bis 1,5 bara.

Der Druck in der Mischzone liegt vorzugsweise in einem Bereich von 0,05 bis 3 bara und die Temperatur in der Mischzone in einem Bereich von 200 bis 400 °C. Der Druck und die Temperatur in der Mischzone ergeben sich dabei aus dem Druck und der Temperatur des in die Mischzone einströmenden Phosgens und Amins. Das in die Mischzone einströmende Phosgen wird vorzugsweise auf eine Temperatur im Bereich von 250 bis 450 °C erwärmt.

Zur Erwärmung des Phosgens und des Amins und zur gleichzeitigen Verdampfung des Amins wird zum Beispiel eine elektrische Beheizung oder eine direkte oder indirekte Aufheizung durch Verbrennung eines Brennstoffs eingesetzt. Als Brennstoffe werden üblicherweise Brenngase, beispielsweise Erdgas, eingesetzt. Durch die Absenkung der Siedetemperatur des Amins ist jedoch auch eine Beheizung, zum Beispiel durch Wasserdampf, möglich. In Abhängigkeit von der Siedetemperatur des Amins wird hierbei der Druck des Wasserdampfes ausgewählt. Ein geeigneter Dampfdruck des Wasserdampfes liegt zum Beispiel im Bereich von 40 bis 100 bar. Daraus ergibt sich eine Temperatur des Wasserdampfes im Bereich von 250 bis 311 °C.

Im Allgemeinen ist es erforderlich, das Amin mehrstufig auf die Reaktionstemperatur zu erwärmen. Im Allgemeinen wird das Amin hierzu zunächst vorerwärmt, dann verdampft und anschließend überhitzt. Im Allgemeinen erfordert die Verdampfung die längsten Verweilzeiten und führt so zu Zersetzungen des Amins. Um dies zu minimieren, ist eine Verdampfung bei niedrigeren Temperaturen, wie es sich z.B. durch den niedrigeren Druck ergibt, vorteilhaft. Um nach der Verdampfung das verdampfte Amin auf Reaktionstemperatur zu überhitzen, ist im Allgemeinen eine Beheizung mit Wasserdampf nicht ausreichend. Zur Überhitzung wird daher üblicherweise eine elektrische Beheizung oder eine direkte oder indirekte Aufheizung durch Verbrennung eines Brennstoffs eingesetzt.

Im Unterschied zur Verdampfung des Amins erfolgt die Verdampfung des Phosgens im Allgemeinen bei deutlich niedrigeren Temperaturen. Aus diesem Grund kann zur Verdampfung des Phosgens im Allgemeinen Wasserdampf eingesetzt werden. Jedoch ist auch die notwendige Überhitzung des Phosgens, um dieses auf Reaktionstemperatur zu erwärmen, im Allgemeinen nur durch eine elektrische Beheizung oder eine direkte oder indirekte Aufheizung durch Verbrennung eines Brennstoffs möglich.

Im Diffusor wird die Temperatur des Reaktionsgemischs auf Reaktionstemperatur erhöht. Gleichzeitig erfolgt auch eine Druckerhöhung auf Reaktionsdruck. Die Druck- und Temperaturerhöhung ergibt sich dabei aus der geometrischen Gestaltung des Diffusors. In Abhängigkeit vom eingesetzten Amin wird die Temperatur im Diffusor vorzugsweise auf 250 bis 450 °C und der Druck auf 0,5 bis 3 bara erhöht. Besonders bevorzugt liegt die Temperatur, auf die das Reaktionsgemisch im Diffusor erhöht wird, im Bereich von 300 bis 400 °C. Der Druck, auf den das Reaktionsgemisch komprimiert wird, liegt besonders bevorzugt im Bereich von 0,8 bis 3,0 bara.

Das Phosgen wird im ersten Einlass, vorzugsweise in der zentralen Düse, alternativ jedoch auch im Ringspalt, auf Schallgeschwindigkeit oder Überschallgeschwindigkeit, zum Beispiel mit Hilfe einer Laval-Düse, beschleunigt. Hierdurch tritt das Phosgen aus der Düse mit Schallgeschwindigkeit oder Überschallgeschwindigkeit aus. In Abhängigkeit von den Druckverhältnissen in der Mischzone kann das Phosgen nach Austritt aus der zentralen Düse auf höhere Geschwindigkeiten nachexpandieren.

Die Schallgeschwindigkeit eines Gases ist im Allgemeinen abhängig von der Temperatur. Je höher die Temperatur ist, umso größer ist auch die Schallgeschwindigkeit. Vorteilhafterweise liegt die Temperatur des Phosgens, das über die zentrale Düse in die Mischzone einströmt im Bereich von 250 bis 450 °C. Damit liegt die Schallgeschwindigkeit des Phosgens am Düsenaustritt vorzugsweise im Bereich von 210 bis 254 m/s. Die Eintrittsgeschwindigkeit des Phosgens in die Mischzone entspricht vorzugsweise mindestens der Schallgeschwindigkeit. Durch eine geeignete geometrische Gestaltung der Phosgenzuführung, z.B. in Form einer Laval-Düse, kann eine Beschleunigung über die Schallgeschwindigkeit hinaus, d.h. auf Überschallgeschwindigkeit, erfolgen.

Die hohe Geschwindigkeit in der Mischzone führt zu verminderten Temperaturen und einem niedrigeren Druck. Damit erfolgt die Vermischung des Phosgens mit dem Amin bei geringeren Temperaturen und Drücken und somit bei einer niedrigen Reaktionsgeschwindigkeit. Hierdurch lassen sich Nebenreaktionen im unvermischten Zustand reduzieren und höhere Selektivitäten erzielen. Erst nach vollständiger Vermischung mit Eintritt in den Diffusor wird durch den Aufbau von Druck und Temperatur die Reaktionsgeschwindigkeit beschleunigt. Die in der Mischzone auftretenden hohen Geschwindigkeiten vermindern zudem die Ablagerungsneigung von gegebenenfalls gebildeten Feststoffen, beispielsweise Harnstoffen, Uretdionen, Isocyanuraten, Hydrochloriden oder Birureten, so dass auch bei der Laufzeit der Anlage Vorteile erhalten werden.

Durch die Austrittsgeschwindigkeit des Phosgens aus der zentralen Düse oder alternativ aus dem Ringspalt ergibt sich in Abhängigkeit von der Geometrie der zentralen Düse und des Ringspalts vorzugsweise eine Geschwindigkeit, mit der das Amin aus dem Ringspalt beziehungsweise aus der zentralen Düse austritt, im Bereich von 30 bis 160 m/s. Besonders bevorzugt tritt das Amin mit einer Geschwindigkeit im Bereich von 50 bis 150 m/s aus dem Ringspalt beziehungsweise aus der zentralen Düse aus.

Um eine vollständige Vermischung des Phosgens mit dem Amin in der Mischzone zu erzielen, ist es bevorzugt, wenn das Verhältnis von Länge zu Durchmesser der Mischzone (UD-Verhältnis) größer ist als 2. Gleichzeitig ist es bevorzugt, wenn das UD-Verhältnis nicht größer ist als 10, damit möglichst schnell nach der vollständigen Vermischung von Amin und Phosgen der Druck und die Temperatur und damit die Reaktionsgeschwindigkeit durch Eintritt in den Diffusor erhöht werden. Besonders bevorzugt liegt das L/D-Verhättnis im Bereich von 4 bis 7.

Ein schnelles Durchmischen von Phosgen und Amin in der Mischzone und die erwünschte Temperatur- und Druckabsenkung wird dadurch erzielt, dass die mittlere Geschwindigkeit in der Mischzone vorzugsweise im Bereich von 50 bis 260 m/s liegt. Besonders bevorzugt liegt die mittlere Geschwindigkeit in der Mischzone im Bereich von 100 bis 250 m/s. Diese Geschwindigkeit wird insbesondere durch die Austrittsgeschwindigkeit des Phosgens aus der zentralen Düse erzielt. In dem sich an die Mischzone anschließenden Diffusor wird die Geschwindigkeit des Reaktionsgemisches verringert. Hierdurch steigen Druck und Temperatur an. Zur Erzielung der für die Phosgenierung des Amins erforderlichen Temperatur liegt die Geschwindigkeit am Austritt des Diffusors vorzugsweise im Bereich von 1 bis 50 m/s. Besonders bevorzugt liegt die Geschwindigkeit des Reaktionsgemisches am Austritt aus dem Diffusor im Bereich von 2 bis 30 m/s.

An den Diffusor schließt sich ein üblicher Reaktor an, wie er zur Durchführung der Phosgenierung eines Amins zur Herstellung von Isocyanaten eingesetzt wird. Im Allgemeinen eingesetzte Reaktoren sind Rohrreaktoren. Der Durchmesser des Rohrreaktors entspricht dabei vorzugsweise dem Austrittsdurchmesser des Diffusors.

Um die Verringerung der Geschwindigkeit im Diffusor und damit den notwendigen Druck und Temperaturaufbau zu erzielen, weist der Diffusor vorzugsweise einen Öffnungswinkel im Bereich von 4 bis 20 ° auf. Das Verhältnis der Länge des Diffusors, bezogen auf den Durchmesser am Diffusoraustritt, liegt vorzugsweise im Bereich von 3 bis 14. Besonders bevorzugt weist der Diffusor einen Öffnungswinkel im Bereich von 6 bis 16 ° auf und das Verhältnis der Länge des Diffusors, bezogen auf den Durchmesser am Diffusoraustritt, liegt im Bereich von 3,5 bis 9,5.

Zum Erzielen einer schnellen Durchmischung von Phosgen und Amin und um den notwendigen Druckabfall im zweiten Einlass, durch den das Amin zugegeben wird, zu erzielen, ist es bevorzugt, wenn das Verhältnis des Öffnungsquerschnitts des zweiten Einlasses zum Öffnungsquerschnitt des ersten Einlasses, durch den das Phosgen zugegeben wird, im Bereich von 1 bis 20 liegt. Bevorzugt liegt das Verhältnis des Öffnungsquerschnitts des zweiten Einlasses zum Öffnungsquerschnitt des ersten Einlasses, durch den das Phosgen zugegeben wird, im Bereich von 1,2 bis 15. Ganz besonders bevorzugt liegt das Verhältnis im Bereich von 1,5 bis 10.

Durch das Verhältnis vom Öffnungsquerschnitt des Ringspalts zum Öffnungsquerschnitt der Düse ergibt sich auch das Verhältnis an Phosgen zu Amin im Reaktionsgemisch. Der Anteil an Amin ist dabei abhängig von der Geschwindigkeit, mit der das Amin durch den Ringspalt in die Mischzone gesaugt wird und weiterhin vom Querschnitt des Ringspalts. Je größer der Querschnitt bzw. je höher die Geschwindigkeit des Amins ist, umso größer ist der Anteil an Amin. Um nur den für die Reaktion notwendigen Anteil an Amin durch den Ringspalt in die Mischzone zuzuführen, ist des möglich, das Amin mit einem Inertgas zu mischen. Insbesondere ist es zur Vermeidung der Bildung von Nebenprodukten bevorzugt, Phosgen im Überschuss zuzuführen. Durch den Anteil an Inertgas im Amin lässt sich die Menge des zugeführten Amins bei vorgegebener Geometrie von zentraler Düse und Ringspalt und vorgegebener Geschwindigkeit und damit Volumenstrom des Phosgens einstellen. Inertmedien, die zugegeben werden, sind solche, die im Reaktionsraum gasförmig vorliegen und nicht mit den im Reaktionsverlauf auftretenden Verbindungen reagieren. Als Inertmedium können zum Beispiel Stickstoff, Edelgase wie Helium oder Argon, Aromaten wie Chlorbenzol, Chlortoluol, o-Dichlorbenzol, Toluol, Xylol, Chlornaphthalin, Decahydronaphthalin, Kohlenstoffdioxid oder Kohlenstoffmonoxid eingesetzt werden. Bevorzugt werden jedoch Stickstoff und/oder Chlorbenzol als Inertmedium verwendet.

Alternativ ist es jedoch auch möglich, zum Beispiel um einen zu großen Überschuss an Phosgen zu vermeiden, das Inertmedium dem Phosgen zuzumischen.

Im Allgemeinen wird das Inertmedium in einer Menge zugesetzt, dass das Verhältnis der Gasvolumina von Inertmedium zu Amin bzw. zu Phosgen mehr als 0,0001 bis 30, bevorzugt mehr als 0,01 bis 15 und besonders bevorzugt mehr als 0,1 bis 5 beträgt.

Amine, die zur Herstellung von Isocyanaten eingesetzt werden können, sind Monoamine, Diamine, Triamine oder höherwertige Amine. Bevorzugt werden Monoamine oder Diamine eingesetzt. Entsprechend des eingesetzten Amins ergeben sich die korrespondierenden Monoisocyanate, Diisocyanate, Triisocyanate oder höherwertigen Isocyanate. Bevorzugt werden Monoisocyanate oder Diisocyanate mit dem erfindungsgemäßen Verfahren hergestellt.

Die Amine und Isocyanate können aliphatisch, cycloaliphatisch oder aromatisch sein. Bevorzugt sind die Amine aliphatisch oder cycloaliphatisch, besonders bevorzugt aliphatisch.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

(Cyclo)aliphatische Isocyanate stehen im Rahmen dieser Anmeldung kurz für cycloaliphatische und/oder aliphatische Isocyanate.

Beispiele für aromatische Mono- und Diisocyanate sind bevorzugt solche mit 6 bis 20 C-Atomen, beispielsweise Phenylisocyanat, monomeres 2,4'- und/oder 4,4'-Methylen-di(phenylisocyanat) (MDI), 2,4- und/oder 2,6-Toluylendiisocyanat (TDI) und 1,5- oder 1,8-Naphthyldiisocyanat (NDI).

Diisocyanate sind bevorzugt (cyclo)aliphatische Diisocyanate, besonders bevorzugt (cyclo)aliphatische Diisocyanate mit 4 bis 20 C-Atomen.

Beispiele für übliche Diisocyanate sind aliphatische Diisocyanate, wie 1,4-Tetramethylendiisocyanat, Hexamethylendiisocyanat (1,6-Diisocyanatohexan), 1,8-Octamethylendiisocyanat, 1,10-Decamethylendiisocyanat, 1,12-Dodecamethylendiisocyanat, 1,14-Tetradecamethylendiisocyanat, 1,5-Diisocyanatopentan, Neopentandiisocyanat, Derivate des Lysindiisocyanates, Tetramethylxylylendiisocyanat (TMXDI), Trimethylhexandiisocyanat oder Tetramethylhexandiisocyanat, sowie 3 (bzw. 4)-, 8 (bzw. 9)-Bis(isocyanatomethyl)-tricyclo[5.2.1.0^{2.6}]decan-Isomerengemische, sowie cycloaliphatische Diisocyanate, wie 1,4-, 1,3- oder 1,2-Diisocyanatocyclohexan, 4,4'- oder 2,4'-Di(isocyanatocyclo-hexyl)methan, 1-Isocyanato-3,3,5-trimethyl-5-(isocyanatomethyl)cyclohexan (Isophorondiisocyanat), 1,3- oder 1,4-bis(lsocyanatomethyl)cyclohexan, 2,4-, oder 2,6-Diiso-cyanato-1-methylcyclohexan.

Bevorzugt sind 1,6-Diisocyanatohexan, 1-Isocyanato-3,3,5-trimethyl-5-(isocyanatomethyl)cyclohexan, 4,4'-Di(isocyanatocyclohexyl)methan und Toluylendiisocyanat-Isomerengemische. Besonders bevorzugt sind 1,6-Diisocyanatohexan, 1-Isocyanato-3,3,5-trimethyl-5-(isocyanatomethyl)cyclohexan und 4,4'-Di(isocyanatocyclo-hexyl)methan.

Amine, die bei dem erfindungsgemäßen Verfahren zur Reaktion zu den korrespondierenden Isocyanaten eingesetzt werden, sind solche, bei denen das Amin, die korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens zwei Mol%, besonders bevorzugt zu höchstens 1 Mol% und ganz besonders bevorzugt zu höchstens 0,5 Mol% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,6-Diaminohexan, 1,5-Diaminopentan, 1,3-bis(Aminomethyl)cyclohexan, 1-Amino-3,3,5-trimethyl-5-Aminomethylcyclohexan (IPDA) und 4,4'-Diaminodicyclohexylmethan. Bevorzugt verwendet wird 1,6-Diaminohexan (HDA).

Ebenfalls können für das erfindungsgemäße Verfahren aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (Mol/Mo)-Gemisch, Diaminobenzol, 2,6-Xylidin, Naphthyldiamin (NDA) und 2,4'- oder 4,4'-Methylen (diphenylamin) (MDA) oder Isomerengemische davon. Bevorzugt sind unter diesen die Diamine, besonders bevorzugt sind 2,4- und/oder 2,6-TDA oder 2,4'- und/oder 4,4'-MDA.

Zur Herstellung von Monoisocyanaten können ebenfalls aliphatische, cycloaliphatische oder aromatische Amine, üblicherweise Monoamine, eingesetzt werden. Als aromatisches Monoamin ist insbesondere Anilin bevorzugt.

Bei der Gasphasenphosgenierung ist es anzustreben, dass die im Reaktionsverlauf auftretenden Verbindungen, also Edukte (Amin und Phosgen), Zwischenprodukte (insbesondere die intermediär entstehenden Mono- und Dicarbamoylchloride), Endprodukte (Diisocyanat), sowie gegebenenfalls zudosierte inerte Verbindungen, unter den Reaktionsbedingungen in der Gasphase verbleiben. Sollten diese oder andere Komponenten sich aus der Gasphase zum Beispiel an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für auftretende Aminhydrochloride, die sich aus freien Aminogruppen und Chlorwasserstoff bilden, da die resultierenden Aminhydrochloride leicht ausfallen und nur schwer wieder verdampfbar sind.

Neben dem Einsatz eines Rohrreaktors ist es auch möglich, im Wesentlichen quaderförmige Reaktionsräume, beispielsweise Plattenreaktoren zu verwenden. In diesem Fall ist der Diffusor so ausgestaltet, dass der Austrittsquerschnitt des Diffusors dem Querschnitt des Reaktors entspricht. Die Länge des Reaktors wird unabhängig von der Querschnittsform vorzugsweise so gewählt, dass innerhalb des Reaktors das Amin im Wesentlichen vollständig zum korrespondierenden Isocyanat umgesetzt wird. Die Kontaktzeit des Reaktionsgemisches beträgt dazu im Allgemeinen zwischen 0,001 s und weniger als 5 s, bevorzugt 0,01 bis 3 s, besonders bevorzugt 0,015 bis weniger als 2 s. Im Fall der Umsetzung von (Cyclo)aliphatischen Aminen kann die mittlere Kontaktzeit besonders bevorzugt von 0,015 bis 1,5 s, insbesondere von 0,015 bis 0,5 s, speziell von 0,02 bis 0,1 s und oft von 0,025 bis 0,05 s betragen. Die Kontaktkzeit wird durch die Geschwindigkeit des Reaktionsgemisches im Reaktor und die Reaktorlänge eingestellt.

Unter mittlerer Kontaktzeit wird dabei die Zeitspanne vom Beginn der Vermischung der Edukte bis zum Verlassen des Reaktionsraumes in einer Aufarbeitungsstufe verstanden. In einer bevorzugten Ausführungsform ist die Strömung im Reaktor des erfindungsgemäßen Verfahrens durch eine Bodenstein-Zahl von mehr als 10, bevorzugt mehr als 100 und besonders bevorzugt mehr als 500 charakterisiert.

Aufgrund der hohen Geschwindigkeiten, die auch am Austritt des Diffusors noch vorherrschen, ist die Strömung im Reaktor üblicherweise turbulent. Hierdurch werden enge Verweilzeitverteilungen mit geringer Standardabweichung von meist nicht mehr als 6 Prozent und eine gute Vermischung erreicht. Eine Verengung im Reaktor, die zudem verstopfungsanfällig ist, ist nicht notwendig. Jedoch kann es sinnvoll sein, in den Reaktor zum Beispiel Strömungsvergleichmäßiger einzubauen, wie sie dem Fachmann bekannt sind.

Wenn es erforderlich ist, kann das Reaktionsvolumen temperiert werden. Üblicherweise erfolgt die Temperierung über den Außenmantel des Reaktors. Um Produktionsanlagen mit hoher Anlagenkapazität zu bauen, ist es möglich, mehrere Reaktorrohre parallel zu schalten. In diesem Fall erfolgt die Zufuhr in jedes einzelne Reaktorrohr über die zentrale Düse mit dem umgebenden Ringraum und der nachfolgenden Mischzone und Diffusor. Die einzelnen Rohre sind dabei üblicherweise als Rohrbündel in einem Rohrbündelreaktor angeordnet. Neben einer Temperierung der Reaktoren ist es aber auch möglich, die Reaktion beispielsweise adiabat durchzuführen. In diesem Fall werden die einzelnen Reaktoren üblicherweise thermisch isoliert.

An den Reaktor schließt sich üblicherweise eine Aufbereitungseinrichtung an. Im Allgemeinen wird das gasförmige Gemisch, das den Reaktor verlässt, vorzugsweise bei Temperaturen von mehr als 130 °C mit einem Lösungsmittel gewaschen. Als Lösungsmittel sind bevorzugt Kohlenwasserstoffe, die gegebenenfalls mit Halogenatomen substituiert sind, geeignet. Geeignete Lösungsmittel sind zum Beispiel Hexan, Benzol, 1,3,5-Trimethylbenzol, Nitrobenzol, Anisol, Chlorbenzol, Chlortoluol, o-Dichlorbenzol, Trichlorbenzol, Diethylisophtalat (DEIP), Tetrahydrofuran (THF), Dimethylformamid (DMF), Xylol, Chlornaphthalin, Decahydronaphthalin und Toluol. Als Lösungsmittel wird besonders bevorzugt Monochlorbenzol eingesetzt. Als Lösungsmittel kann jedoch auch beispielsweise das Isocyanat eingesetzt werden. Bei der Wäsche wird das Isocyanat selektiv in die Waschlösung überführt. Anschließend werden das verbleibende Gas und die erhaltene Waschlösung bevorzugt mittels Rektifikation in Isocyanat, Lösungsmittel, Phosgen und Chlorwasserstoff aufgetrennt.

Das Waschen des den Reaktor verlassenden Gasgemisches erfolgt vorzugsweise in einem Waschturm, wobei aus dem gasförmigen Gemisch das gebildete Isocyanat durch Kondensation in einem inerten Lösungsmittel abgetrennt wird, während überschüssiges Phosgen, Chlorwasserstoff und gegebenenfalls das Inertmedium die Aufarbeitungsvorrichtung gasförmig durchlaufen. Bevorzugt wird dabei die Temperatur des inerten Lösungsmittels oberhalb der Lösungstemperatur des zum Amin gehörigen Carbamylchlorids im gewählten Waschmedium gehalten. Besonders bevorzugt wird dabei die Temperatur des inerten Lösungsmittels oberhalb der Schmelztemperatur des zum Amin gehörigen Carbamylchlorids gehalten.

Die Wäsche kann in jeder beliebigen, dem Fachmann bekannten Vorrichtung durchgeführt werden. So eignen sich zum Beispiel Rührbehälter oder andere herkömmliche Apparaturen, beispielsweise Kolonnen oder Mixer-Settler-Apparaturen.

Das Waschen und die Aufarbeitung des den Reaktor verlassenden Gemischs erfolgt dabei im Allgemeinen wie beispielsweise in WO-A 2007/028715 beschrieben.

Nachfolgend wird die Erfindung anhand einer Zeichnung näher erläutert.

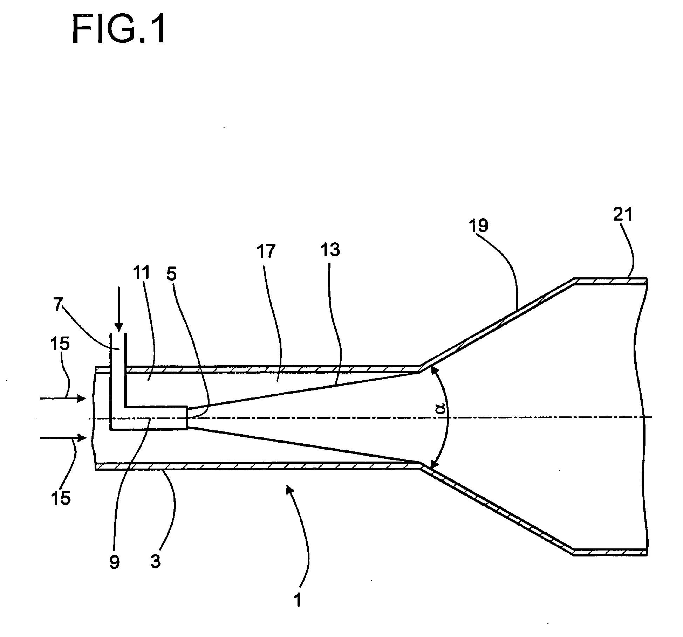

Die einzige Figur zeigt schematisch einen Ejektor zur Durchführung des erfindungsgemä-βen Verfahrens.

Ein Ejektor 1 umfasst ein Rohr 3, in das eine zentrale Düse 5 mündet. Das Rohr 3 umschließt einen Zulauf 7 zur zentralen Düse 5. Der Zulauf 7 ist rohrförmig gestaltet und weist einen Abschnitt 9 auf, der entlang der Achse des Rohres 3 verläuft. Auf diese Weise wird zwischen dem Abschnitt 9 des Zulaufs 7 und dem den Abschnitt 9 des Zulaufs 7 umgebenden Rohr 3 ein Ringspalt 11 ausgebildet.

Über den Zulauf 7 wird dem Ejektor 1 Phosgen zugeführt. Das Phosgen tritt aus dem Zulauf 7 über die zentrale Düse 5 mit einer hohen Geschwindigkeit aus. Vorzugsweise tritt das Phosgen mit Schallgeschwindigkeit aus der zentralen Düse 5 aus.

Das aus der zentralen Düse 5 austretende Phosgen bildet einen sich aufweitenden Strahl 13, wodurch Medium aus der Umgebung mitgerissen wird. Hierdurch entsteht ein Unterdruck im Ringspalt 11. Durch den am Ringspalt 11 entstehenden Unterdruck wird ein Amin beispielsweise aus einem Vorratsbehälter, der das Amin enthält, angesaugt, strömt durch den Ringspalt 11 und gelangt in eine Mischzone 17, die sich an die zentrale Düse 5 im Rohr 3 anschließt. Der Zulauf des Amins ist in der Figur durch Pfeile 15 dargestellt.

In der Mischzone 17, die sich an die zentrale Düse 5 im Rohr 3 anschließt, vermischen sich das aus der zentralen Düse 5 ausströmende Phosgen und das vom Phosgen aus dem Ringspalt 11 mitgerissene Amin zu einem Reaktionsgemisch. Aufgrund der hohen Geschwindigkeit des Phosgens ist die Strömung in der Mischzone 17 turbulent und es erfolgt eine schnelle Vermischung von Amin und Phosgen.

Wie zuvor beschrieben, können das Amin und/oder das Phosgen gegebenenfalls ein Inertmedium enthalten. Bevorzugt als Inertmedium werden Stickstoff oder Chlorbenzol eingesetzt.

Aufgrund des Unterdrucks im Ringspalt 11 verdampft das Amin bereits bei Temperaturen unterhalb der bevorzugten Reaktionstemperatur. Hierdurch wird die Reaktionsgeschwindigkeit des Amins mit dem Phosgen in der Mischzone 17 reduziert und es wird weniger Isocyanat gebildet. Die Menge an Nebenprodukten, die zum Beispiel durch Reaktion des Isocyanats mit dem Amin gebildet werden kann, nimmt dadurch ebenfalls ab.

An die Mischzone 17 schließt sich ein Diffusor 19 an. Im Diffusor 19 nimmt der Strömungsquerschnitt im Allgemeinen konstant zu. Hierzu weist der Diffusor 19 einen Öffnungswinkel α im Bereich von 4° bis 20°, bevorzugt von 6° bis 16 ° auf.

Im Diffusor 19 nimmt die Geschwindigkeit des Reaktionsgemisches ab. Gleichzeitig steigt der Druck und mit dem Druck auch die Temperatur. Die Länge des Diffusors 19 wird vorzugsweise so gewählt, dass am Diffusoraustritt die für eine hohe Reaktonsgeschwindigkeit erforderliche Reaktionstemperatur und der entsprechende Druck herrschen.

An den Diffusor 19 schließt sich ein Reaktor 21 an, in dem die Umsetzung des Amins mit dem Phosgen zum Isocyanat erfolgt. Der Reaktor ist dabei vorzugsweise als Rohrreaktor ausgebildet.

An den Reaktor kann sich eine Aufbereitungseinheit für das das Isocyanat enthaltende Produktgemisch anschließen.

### Beispiel

In einer erfindungsgemäßen Injektordüse werden 1,75 kg/h Toluylendiamin und 8,5 kg/h Phosgen gemischt. Das Toluylendiamin liegt als 80:20 Isomerengemisch aus 2,4- und 2,6-TDA vor. Die Reaktion wird bei einer Starttemperatur von 400°C und einem Druck von 2 bara durchgeführt.

Das Phosgen wird über eine zentrale Düse mit einem Durchmesser von 1,6 mm zugeführt und mündet in einem Mischrohr mit einem Innendurchmesser von 2,9 mm und einem UD-Verhältnis von 6. Der Eintrittsquerschnitt für das Amin beträgt 4,6 mm². Der notwendige Vordruck des Phosgens beträgt 4,4 bara. Daraus ergibt sich eine rechnerische Geschwindigkeit im Mischrohr von 190 m/s. Das Phosgen tritt mit Schallgeschwindigkeit von 245 m/s aus der Düse aus. Durch die Beschleunigung des Phosgens kühlt dieses auf 360°C ab. Der sich einstellende Druck zu Beginn der Mischzone und in der Ansaugleitung des Amins beträgt damit ca. 1,25 bara. Die Siedetemperatur des Amins kann aufgrund der Druckabsenkung von 2 bara auf 1,25 bara von 315°C auf 295°C gesenkt werden. Aufgrund der verringerten Partialdrücke der Edukte ist die Reaktionsgeschwindigkeit am Anfang der Mischzone um mehr als 60% niedriger als zu den Bedingungen, die im Reaktor herrschen.

In dem sich an die Mischzone anschließenden Diffusor, der einen Öffnungswinkel von 12° aufweist, erweitert sich der Durchmesser von 2,9 mm auf 18 mm. Die Temperatur des Reaktionsgemisches, das den Diffusor durchströmt, erhöht sich auf 400°C und der Druck auf 2 bara.

### Bezugszeichenliste

- 1: Ejektor
- 3: Rohr
- 5: zentrale Düse
- 7: Zulauf
- 9: Abschnitt des Zulaufs 7
- 11: Ringspalt
- 13: aufweitender Strahl
- 15: Aminzulauf
- 17: Mischzone
- 19: Diffusor
- 21: Reaktor

- α: Öffnungswinkel

## Patentansprüche

1. Verfahren zur Herstellung von Isocyanaten durch Umsetzung der korrespondierenden Amine mit Phosgen in der Gasphase, gegebenenfalls in Gegenwart mindestens eines Inertmediums, wobei das Phosgen durch einen ersten Einlass und das Amin durch einen zweiten Einlass eines Ejektors (1) in einen Reaktor (21) geleitet werden, **dadurch gekennzeichnet, dass** der erste Einlass und der zweite Einlass in eine Mischzone (17) münden, in der das Phosgen und das Amin zu einem Reaktionsgemisch gemischt werden und sich an die Mischzone (17) ein Diffusor (19) anschließt, in dem Druck und Temperatur des Reaktionsgemischs aus Phosgen und Amin erhöht werden.

2. Verfahren gemäß Anspruch 1, **dadurch gekennzeichnet, dass** durch die Strömung des Phosgens durch den ersten Einlass im zweiten Einlass und in einem in den zweiten Einlass mündenden Vorlauf des Amins ein Unterdruck erzeugt wird.

3. Verfahren gemäß Anspruch 1 oder 2, **dadurch gekennzeichnet, dass** der Druck im zweiten Einlass und im in den zweiten Einlass mündenden Vorlauf im Bereich von 0,01 bis 3 bara liegt.

4. Verfahren gemäß einem der Ansprüche 1 bis 3, **dadurch gekennzeichnet, dass** der erste Einlass eine zentrale Düse (5) und der zweite Einlass ein die zentrale Düse (5) umgebender Ringspalt (11) des Injektors (1) ist.

5. Verfahren gemäß einem der Ansprüche 1 bis 4, **dadurch gekennzeichnet, dass** der Druck in der Mischzone (17) in einem Bereich von 0,05 bis 3 bara und die Temperatur in der Mischzone (17) in einem Bereich von 200 bis 400°C liegt.

6. Verfahren gemäß einem der Ansprüche 1 bis 5, **dadurch gekennzeichnet, dass** die Temperatur im Diffusor (19) auf 250 bis 450°C und der Druck auf 0,5 bis 3 bara erhöht werden

7. Verfahren gemäß einem der Ansprüche 1 bis 6, **dadurch gekennzeichnet, dass** die Geschwindigkeit, mit der das Phosgen aus der zentralen Düse (5) austritt, Schallgeschwindigkeit oder Überschallgeschwindigkeit ist.

8. Verfahren gemäß einem der Ansprüche 1 bis 7, **dadurch gekennzeichnet, dass** die Geschwindigkeit, mit der das Phosgen aus der zentralen Düse (5) austritt, im Bereich von 200 bis 500 m/s liegt.

9. Verfahren gemäß einem der Ansprüche 1 bis 8, **dadurch gekennzeichnet, dass** die Geschwindigkeit, mit der das Amin aus dem Ringspalt (11) austritt, im Bereich von 30 bis 160 m/s liegt.

10. Verfahren gemäß einem der Ansprüche 1 bis 9, **dadurch gekennzeichnet, dass** das Verhältnis von Länge zu Durchmesser der Mischzone (17) im Bereich von 2 bis 10 liegt.

11. Verfahren gemäß einem der Ansprüche 1 bis 10, **dadurch gekennzeichnet, dass** die mittlere Geschwindigkeit in der Mischzone (17) im Bereich von 100 bis 250 m/s und die mittlere Geschwindigkeit des Reaktionsgemischs am Austritt aus dem Diffusor (19) im Bereich von 1 bis 50 m/s liegt.

12. Verfahren gemäß einem der Ansprüche 1 bis 11, **dadurch gekennzeichnet, dass** der Diffusor (19) einen Öffnungswinkel (α) im Bereich von 4 bis 20° aufweist und das Verhältnis der Länge des Diffusors (19) bezogen auf den Durchmesser des Diffusors (19) am Diffusoraustritt im Bereich von 3 bis 14 liegt.

13. Verfahren gemäß einem der Ansprüche 1 bis 12, **dadurch gekennzeichnet, dass** das Verhältnis des zweiten Einlasses, durch den das Amin zugegeben wird, zum Öffnungsquerschnitt des ersten Einlasses, durch den das Phosgen zugegeben wird, im Bereich von 1 bis 20 liegt.

14. Verfahren gemäß einem der Ansprüche 1 bis 13, **dadurch gekennzeichnet, dass** das Isocyanat ein Monoisocyanat oder Diisocyanat ist.

15. Verfahren gemäß einem der Ansprüche 1 bis 14, **dadurch gekennzeichnet, dass** das eingesetzte Amin 1,6-Diaminohexan, monomeres 2,4'- und/oder 4,4'-Methylen(diphenylamin), 2,4- und/oder 2,6-Toluylendiamin oder 1-Amino-3,3,5-trimethyl-5-Aminomethylcyclohexan ist.

## Claims

1. A process for preparing isocyanates by reacting the corresponding amines with phosgene in the gas phase, if appropriate in the presence of at least one inert medium, the phosgene being passed into a reactor (21) through a first inlet and the amine through a second inlet of an ejector (1), wherein the first inlet and the second inlet open into a mixing zone (17) in which the phosgene and the amine are mixed to give a reaction mixture and the mixing zone (17) is followed downstream by a diffuser (19) in which pressure and temperature of the reaction mixture composed of phosgene and amine are increased.

2. The process according to claim 1, wherein the flow of the phosgene through the first inlet generates a reduced pressure in the second inlet and in a feed of the amine opening into the second inlet.

3. The process according to claim 1 or 2, wherein the pressure in the second inlet and in the feed opening into the second inlet is in the range from 0.01 to 3 bara.

4. The process according to any one of claims 1 to 3, wherein the first inlet is a central nozzle (5) and the second inlet is an annular gap (11) of the injector (1) surrounding the central nozzle (5).

5. The process according to any one of claims 1 to 4, wherein the pressure in the mixing zone (17) is within a range from 0.05 to 3 bara and the temperature in the mixing zone (17) is within a range from 200 to 400°C.

6. The process according to any one of claims 1 to 5, wherein the temperature is increased to from 250 to 450°C and the pressure to from 0.5 to 3 bara in the diffuser (19).

7. The process according to any one of claims 1 to 6, wherein the velocity with which the phosgene leaves the central nozzle (5) is sonic velocity or supersonic velocity.

8. The process according to any one of claims 1 to 7, wherein the velocity with which the phosgene leaves the central nozzle (5) is in the range from 200 to 500 m/s.

9. The process according to any one of claims 1 to 8, wherein the velocity with which the amine leaves the annular gap (11) is in the range from 30 to 160 m/s.

10. The process according to any one of claims 1 to 9, wherein the ratio of length to diameter of the mixing zone (17) is in the range from 2 to 10.

11. The process according to any one of claims 1 to 10, wherein the mean velocity in the mixing zone (17) is in the range from 100 to 250 m/s and the mean velocity of the reaction mixture at the exit from the diffuser (19) is in the range from 1 to 50 m/s.

12. The process according to any one of claims 1 to 11, wherein the diffuser (19) has an opening angle (α) in the range from 4 to 20° and the ratio of the length of the diffuser (19) based on the diameter of the diffuser (19) at the diffuser exit is in the range from 3 to 14.

13. The process according to any one of claims 1 to 12, wherein the ratio of the second inlet through which the amine is added to the opening cross section of the first inlet through which the phosgene is added is in the range from 1 to 20.

14. The process according to any one of claims 1 to 13, wherein the isocyanate is a monoisocyanate or diisocyanate.

15. The process according to any one of claims 1 to 14, wherein the amine used is 1,6-diaminohexane, monomeric 2,4'- and/or 4,4'-methylene(diphenylamine), 2,4- and/or 2,6-tolylenediamine or 1-amino-3,3,5-trimethyl-5-aminomethylcyclohexane.

## Revendications

1. Procédé pour la production d'isocyanates par mise en réaction des amines correspondantes avec du phosgène dans la phase gazeuse, éventuellement en présence d'au moines un milieu inerte, dans lequel on introduit dans un réacteur (21) le phosgène par une première entrée et l'amine par une deuxième entrée d'un injecteur (1), **caractérisé en ce que** la première entrée et la deuxième entrée débouchent dans une zone de mélange (17), dans laquelle le phosgène et l'amine sont mélangés en un mélange réactionnel et à la zone de mélange (17) fait suite un diffuseur (19), dans lequel sont augmentées la pression et la température du mélange réactionnel constitué de phosgène et d'amine.

2. Procédé selon la revendication 1, **caractérisé en ce que** par l'écoulement du phosgène à travers la première entrée est engendrée une dépression dans la deuxième entrée et dans un conduit d'alimentation de l'amine débouchant dans la deuxième entrée.

3. Procédé selon la revendication 1 ou 2, **caractérisé en ce que** la pression dans la deuxième entrée et dans le conduit d'alimentation débouchant dans la deuxième entrée se situe dans la plage de 0,01 à 3 bars (absolue).

4. Procédé selon l'une quelconque des revendications 1 à 3, **caractérisé en ce que** la première entrée est une buse centrale (5) et la deuxième entrée est un espace annulaire (11) de l'injecteur (1), entourant la buse centrale (5).

5. Procédé selon l'une quelconque des revendications 1 à 4, **caractérisé en ce que** la pression dans la zone de mélange (17) se situe dans une plage de 0,05 à 3 bars (absolue) et la température dans la zone de mélange (17) se situe dans une plage de 200 à 400 °C.

6. Procédé selon l'une quelconque des revendications 1 à 5, **caractérisé en ce que** la température dans le diffuseur (19) est augmentée à 250 - 450 °C et la pression à 0,5 - 3 bars (absolue).

7. Procédé selon l'une quelconque des revendications 1 à 6, **caractérisé en ce que** la vitesse à laquelle le phosgène sort de la buse centrale (5), est la vitesse du son ou une vitesse supersonique.

8. Procédé selon l'une quelconque des revendications 1 à 7, **caractérisé en ce que** la vitesse à laquelle le phosgène sort de la buse centrale (5) se situe dans la plage de 200 à 500 m/s.

9. Procédé selon l'une quelconque des revendications 1 à 8, **caractérisé en ce que** la vitesse à laquelle l'amine sort de l'espace annulaire (11) se situe dans la plage de 30 à 160 m/s.

10. Procédé selon l'une quelconque des revendications 1 à 9, **caractérisé en ce que** le rapport de la longueur au diamètre de la zone de mélange (17) se situe dans la plage de 2 à 10.

11. Procédé selon l'une quelconque des revendications 1 à 10, **caractérisé en ce que** la vitesse moyenne dans la zone de mélange (17) se situe dans la plage de 100 à 250 m/s et la vitesse moyenne du mélange réactionnel à la sortie du diffuseur (19) se situe dans la plage de 1 à 50 m/s.

12. Procédé selon l'une quelconque des revendications 1 à 11 **caractérisé en ce que** le diffuseur (19) présente un angle d'ouverture (α) dans la plage de 4 à 20° et le rapport de la longueur du diffuseur (19) au diamètre du diffuseur (19) à la sortie du diffuseur se situe dans la plage de 3 à 14.

13. Procédé selon l'une quelconque des revendications 1 à 12 **caractérisé en ce que** le rapport de la deuxième entrée, par laquelle est introduite l'amine, à la section transversale de l'ouverture de la première entrée, par laquelle est introduit le phosgène, se situe dans la plage de 1 à 20.

14. Procédé selon l'une quelconque des revendications 1 à 13, **caractérisé en ce que** l'isocyanate est un mono-isocyanate ou diisocyanate.

15. Procédé selon l'une quelconque des revendications 1 à 14, **caractérisé en ce que** l'amine utilisée est le 1,6-diaminohexane, la 2,4'- et/ou la 4,4'-méthylène(diphénylamine) monomère, la 2,4- et/ou la 2,6-toluylènediamine ou le 1-amino-3,3,5-triméthyl-5-aminométhylcyclohexane.
